# EUROPEAN PATENT SPECIFICATION

(11) **EP 1 440 696 B1**
(45) Date of publication and mention of the grant of the patent: **14.01.2009**
(21) Application number: 04001072.0
(22) Date of filing: 20.01.2004
(51) Int. Cl.: A61L 9/12, A61L 9/04

(54) **Volatile material packaging body**
Verpackungskörper für flüchtige Stoffe
Corps d' emballage pour les materiaux volatiles

(30) Priority: 22.01.2003 JP 2003013788
(43) Date of publication of application: 28.07.2004
(73) Proprietor: Zexel Valeo Climate Control Corporation, Ohsato-gun, Saitama 360-0193 (JP); Rengo Co., Ltd., Osaka-shi Osaka-fu 553-0007 (JP)
(72) Inventor: Hara, Sinichi, c/o Zexel Valeo Climate Contr. Corp, Ohsato-gun Saitama 360-0193 (JP); Yoshida, Akihiko, Zexel Valeo Climate Contr. Corp., Ohsato-gun Saitama 360-0193 (JP); Kamei, Kiyoshi, c/o Rengo Co., Ltd., Takefu-shi Fukui-ken 915-0011 (JP); Dangami, Yoshiharu, Chuo-ku Tokyo 103-0013 (JP)
(74) Representative: HOFFMANN EITLE

(56) References cited:
- EP-A- 1 093 721
- EP-A- 1 142 959
- US-A- 4 961 493
- US-A- 5 458 244

## Description

### [Technical Field of the Invention]

The present invention relates to a packaging body which allows gradual volatilization of a volatile material.

### Prior Art

There is generally known a bag, which seals therein a volatile material such as an antimicrobial agent, an aromatic substance, or the like and allows gradual volatilization of the volatile material. The bag is comprised of a sheet material, such as a perforated film, nonwoven fabric, water-proof paper, or the like, having gas permeability. For example, Patent Documents 1 and 2 disclose a film having gas permeability, and Patent Documents 3 to 5 disclose a bag of such a type (see below).

For such a bag, there have been proposed a variety of configurations depending on its purpose of use. For example, Patent Document 6 discloses an air filter which is configured to have a pocket for housing the bag in a filter for a car air conditioner. Patent Document 7 discloses a device configured to be installed in a prescribed position of a car.

Besides, as a packaging body making a volatile material volatilizable, there is known one using a resin casing as disclosed in, for example, Patent Documents 8 to 10. Patent Document 8 discloses a configuration in that gel or sol allylisothiocyanate contained in a polymetic material is housed in a casing having a gas permeating section. Patent Document 9 discloses a configuration in that a humidity sensitive base material is disposed on an open portion of a polypropylene casing for housing a volatile material. The volatile material permeates through a humidity sensitive base material to volatilize outside, and its amount permeating through the wall section of the casing is within a range of error. Besides, Patent Document 10 discloses a configuration in that a volatile material sealed in a bag or the like is housed in a casing of which open portion is covered with a lid formed of a gas permeable sheet. The volatile material permeates through the lid formed of the gas permeable sheet to volatilize outside, and its amount permeating through the casing wall section is in a range of error.

US 4,961,493 relates to an aromatic package comprising a liquid or gel type aromatic and a sack made of a gas-permeable but liquid-impermeable film and hermetically receiving the aromatic.

EP 1 093 721 A1 relates to a controlled-release animal-repellent article permitting controlled release of an animal-repellent substance. The article comprises a volatilization control container enclosing a molded body of a base resin incorporated allyl isothiocyanate.
[Patent Document 1]
   Japanese Patent Laid-Open Publication JP-A-10-80949
[Patent Document 2]
   Japanese Patent Laid-Open Publication JP-A-11-99601
[Patent Document 3]
   Japanese Patent Laid-Open Publication JP-A-7-017508
[Patent Document 4]
   Japanese Patent Laid-Open Publication JP-A-10-101022
[Patent Document 5]
   Japanese Patent Laid-Open Publication JP-A-2000-167032
[Patent Document 6]
   Japanese Patent Publication JP 63-075420A
[Patent Document 7]
   Japanese Patent Laid-Open Publication JP-A-10-315757
[Patent Document 8]
   Japanese Patent Laid-Open Publication JP-A-10-158112
[Patent Document 9]
   Japanese Patent Laid-Open Publication JP-A-2002-060302
[Patent Document 10]
   Japanese Patent Laid-Open Publication JP-A-2002-187804

### Problems to be solved by the Invention

It should be noted that the bag having the volatile material sealed therein as described above is desired to volatilize the volatile material stably for a long period. In this connection, an amount of volatilization of the volatile material increases considerably with an increase in temperature, so that, when it is to be disposed within, for example, a car air conditioner or the like, it is required to take some device for the bag. In other words, a sharp increase in amount of volatilization due to an influence of the temperature increase becomes a cause of uselessly decreasing an effective period of volatilizing the volatile material or producing a strong odor, leading to unpleasantness. And, waterproofing is significant in a place where moisture or humidity is high, and resistance to inflammability is also significant if there is a possibility that a heat source becomes close.

The present invention was made in view of the circumstances described above, and it is an object of the present invention to provide a packaging body which excels in efficient volatilization of a volatile material.

### Means for solving the Problems

The invention recited in Claim 1 of the application is a packaging body for a volatile material, comprising a bag, which has in use the volatile material sealed therein and has gas permeability, and a casing which has the bag sealed therein, wherein the casing has its wall section at least partly made of a resin having gas permeability, and the volatile material permeates through the wall section to volatilize outside, wherein X/Y falls in a range of 6,000 to 35,000 when the gas permeable wall section of the casing has a surface area of X mm² and a thickness of Y mm. By configuring in this way, there is obtained a remarkable packaging body efficiently volatilizing the volatile material. The volatilization amount of the volatile material depends on a wall thickness of the casing and a surface area of the casing. Specifically, when the casing has a thick wall section, the volatilization amount reduces, and when it becomes thin, the volatilization amount increases. And, when the casing has an increased surface arena, the volatilization amount increases, and when the surface area becomes small, the volatilization amount reduces. In other words, the packaging body is a packaging body for a volatile material determined by paying attention to the relationship between the wall thickness of the casing and the surface area of the casing so to determine the relationship to have a good balance.

Specifically, by a double structure having the bag, which has the volatile material sealed therein, and the resin casing for housing the bag, a volatilization speed can be adjusted by each of them, so that the volatilization speed is easily controlled, and.the volatile material can be volatilized stably for a long period. Therefore, an effective period of volatilizing the volatile material can be secured satisfactorily.
The packaging body has the bag, which seals the volatile material therein, fully covered with the casing, so that it is excellent in water resistance and flammability resistance, and it is advantageously used for a long period when the packaging body is disposed in a place where moisture or humidity is high or when there is a possibility that a heat source approaches around it.

In a preferred embodiment a volatilization amount per day of the volatile material is 3.8 to 23.0 mg at 30C°. In other words, the packaging body of the invention efficiently volatilizes the volatile material and especially can be used very suitably as a packaging body having a volatile material volatilization amount of 3.8 to 23.0 mg per day.

In a further preferred embodiment, the wall section is made of polypropylene having a thickness of 0.3 to 2.2 mm. Specifically, the preferred embodiment is a packaging body using polypropylene as a material for the casing with its thickness set to 0.3 to 2.2 mm. The thickness is in a very practical range, which is determined considering the gas permeability, formability and the like of polypropylene.

According to another preferred embodiment, the volatile material is allylisothiocyanate (hereinafter called as AIT) or an agent containing it. Specifically, the packaging body according to this embodiment allows efficient volatilization of the volatile material and especially can be used very suitably as a packaging body to volatilize the AIT or a substance containing it.

### [Brief Description of the Drawings]

[Fig. 1] A perspective view showing a packaging body for a volatile material according to an embodiment of the invention.
[Fig. 2] An exploded perspective view showing the packaging body for a volatile material according to the embodiment of the invention.
[Fig. 3] A volatilization amount characteristic graph of the volatile material according to the embodiment of the invention.
[Fig. 4] A concentration characteristic graph of the volatile material within an air conditioner according to the embodiment of the invention.
[Fig. 5] A concentration characteristic graph of the volatile material within the air conditioner according to the embodiment of the invention.
[Fig. 6] An explanatory diagram showing a yearly volatilization amount of the volatile material according to the embodiment of the invention.
[Fig. 7] A graph indicating the results of measuring the number of microorganisms in drain according to the embodiment of the invention.
[Fig. 8] A volatilization amount characteristic graph of the volatile material to X/Y according to the embodiment of the invention.

### [Embodiment of the Invention]

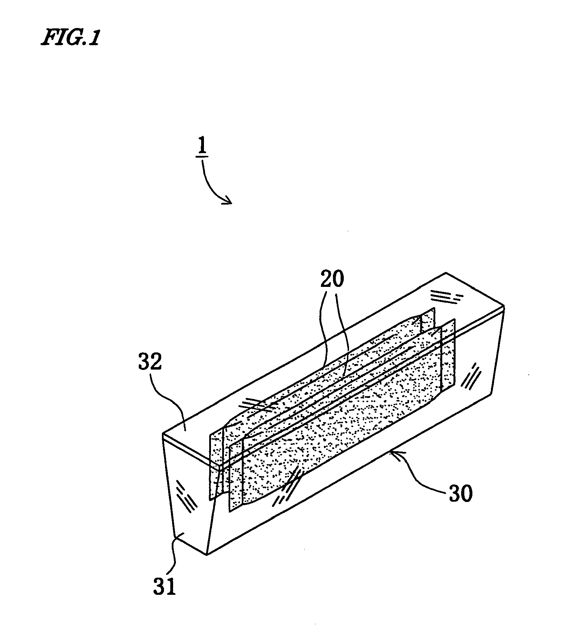

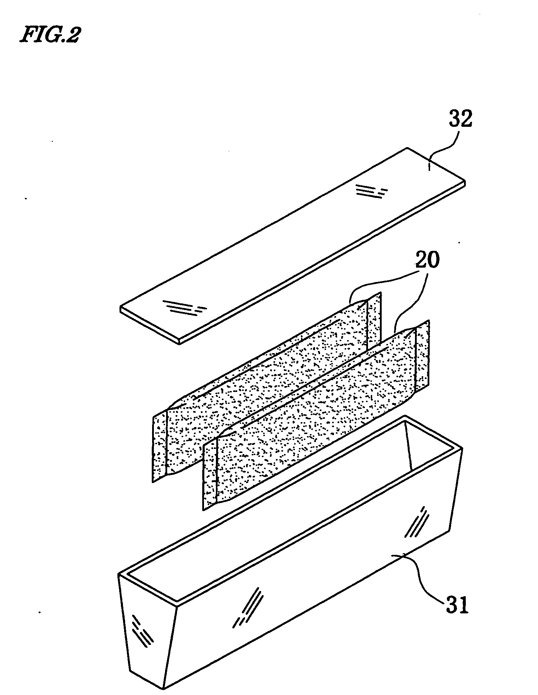

An embodiment of the invention will be described with reference to the drawings. A packaging body 1 for a volatile material according to the embodiment shown in Fig. 1 and Fig. 2 has a bag 20 having gas permeability with the volatile material sealed therein and a casing 30 having the bag 20 sealed therein. The overall casing 30 has its wall section made of a resin having gas permeability, and the volatile material permeates through the wall section of the casing 30 to volatilize outside. In the casing 30 are sealed the prescribed number of bag bodies 20 as required (two in the drawings) . And, the material for the bag is not limited to a particular one but plastic films of polyethylene, cast polypropylene, oriented polypropylene, polyethylene terephthalate and the like are available, and they can be used alone or as a laminate of them. The above-described material may have nonwoven fabric, paper or the like laminated in order to improve bag-production work efficiency.

The casing 30 of this embodiment is formed with an opening of a casing member 31 for housing the bag 20 therein closed with a lid member 32. The casing member 31 and the lid member 32 each is made of an injection-molded member using polypropylene as a material, and the wall section through which the volatile material permeates has a thickness of 0.3 to 2.2 mm.

The casing 30 illustrated is transparent or translucent so that the interior is visible, but it can be made to have a whitish color by mixing polypropylene with talc or can be colored to have a desired color by adding a pigment or the like.

Farther, the casing member 31 and the lid member 32 are mutually joined by ultrasonic welding, and after they are joined, the sealed state of the bag is tested by a water submersion test.

The volatile material sealed in the bag 20 is the AIT which is an antimicrobial agent or a substance containing it, volatilizes first within the casing 30 and gradually permeates through the wall section of the casing 30 to get outside.

In this embodiment, a volatilization amount per day of the volatile material at 30°C is 3.8 to 23.0 mg. When it is assumed that the casing 30 has a surface area of X mm² and the wall section of the casing 30 has a thickness of Y mm, X/Y falls in a range of 6,000 to 35,000.

The packaging body 1 for the volatile material of this embodiment gradually volatilizes the AIT to render its periphery antimicrobial. The AIT concentration in the peripheral air is desirably about 5 to 10 ppm. This packaging body 1 is disposed and used in various types of devices such as an air conditioner, an air cleaner, a washing machine and the like. And, it can also be disposed and used in a kitchen, a closet, a rest room, a bath room, a washroom and the like. Especially, it has an advantage that it is resistant to moisture and humidity and also excels in generality.

The inventors of this application have disposed plural packaging bodies 1 under different conditions within the air conditioner of cars respectively in order to examine in detail the anti-bacterium effect by the packaging body 1 of this embodiment and collected the following data.

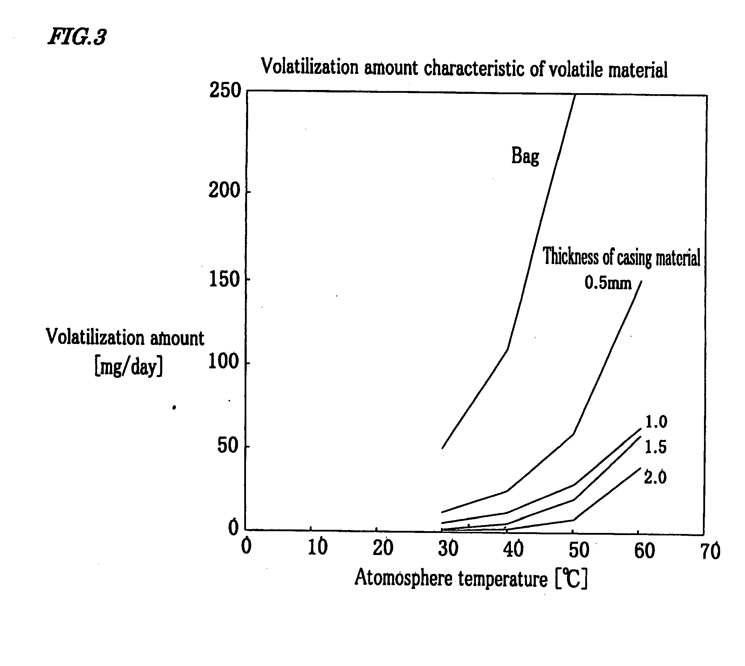

First, plural casings 30 respectively having a different wall thicknesses were prepared and measured for a volatilization amount per day of the individual casings 30 at a different atmosphere temperature. The individual casings 30 each has a surface area of 7500 mm² and a wall thickness of 0.5 mm, 1.0 mm, 1.5 mm and 2.0 mm. Fig. 3 shows the measured results. The bag 20 sealed in each casing 30 comprises a laminate of a 40-µ m oriented polypropylene film and a 30-µ m polyethylene film and has a surface area of 6200 mm². Fig. 3 also shows as a control group the measured results of the volatilization amount without sealing the bag 20 within the casing 30. It is considered from the measured results that the packaging body 1 disposed within the air conditioner is optimum when the casing 30 has a wall thickness of 0.5 to 1.0 mm in view of a relationship between the volatilization amount per day and the atmosphere temperature if the surface area is about 7500 mm².

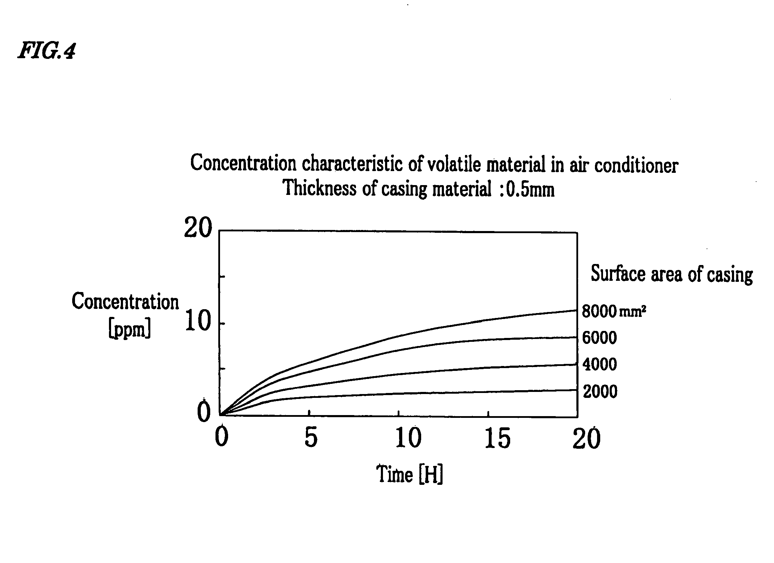

Then, plural casings 30 respectively having a wall thickness of 0.5 mm and a different surface area were prepared, and a change in AIT concentration around the individual casings 30 with time was measured. The casings 30 respectively have surface areas of 2,000 mm², 4,000 mm², 6,000 mm² and 8,000 mm². Fig. 4 shows the measured results. It is considered from the measured results that, when the casing 30 has a wall thickness of 0.5 mm, it is desirable that the casing 30 has a surface area of 4,000 mm² or more in view of the relationship with the AIT concentration.

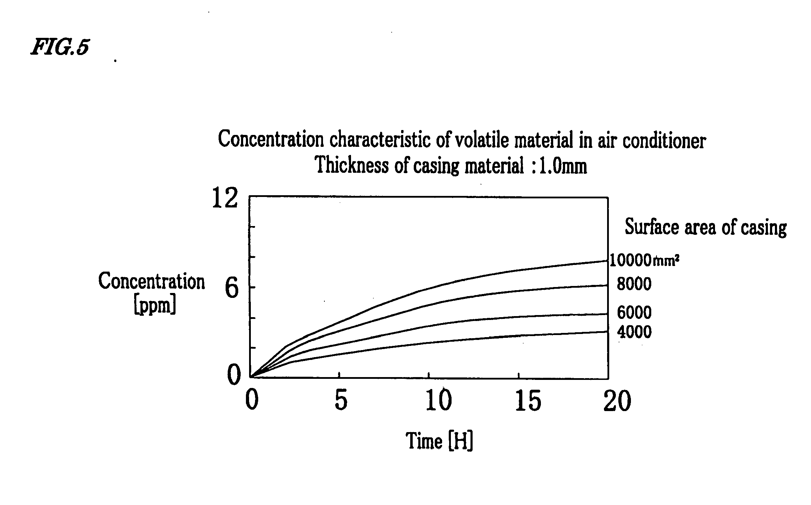

Similarly, plural casings 30 respectively having a wall thickness of 1.0 mm and a different surface area were prepared and measured for a change in AIT concentration around each casing 30 with time. The casings 30 respectively have a surface area of 4,000 mm², 6,000 mm², 8,000mm² and 10,000 mm². Fig. 5 shows the measured results. It is considered from the measured results that, when the casing 30 has a wall thickness of 1.0 mm, the casing 30 desirably has a surface area of 8,000mm² or more in view of the relationship with the AIT concentration.

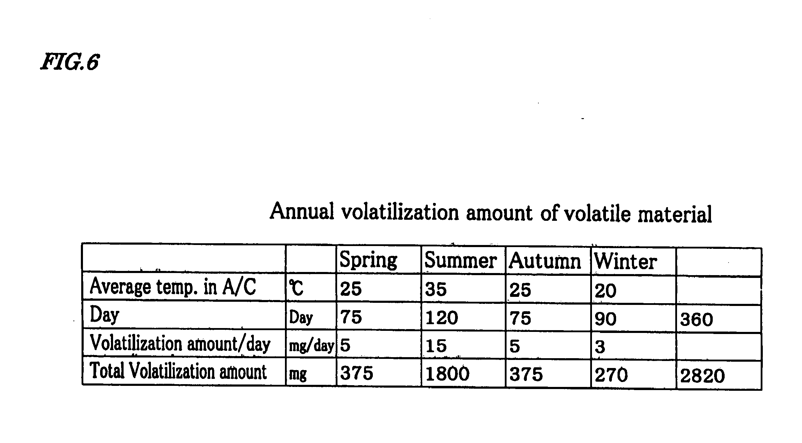

Besides, the inventors of the present application measured a yearly volatilization amount of the volatile material based on a change in temperature depending on seasons. Especially, the microorganisms tend to propagate actively in the summer time, so that it is necessary to have a volatilization amount per day of the AIT greater than that in other seasons in order to secure sufficient antibacterial capability. Fig. 6 shows the measured results. The used packaging body 1 has the casing 30 with a wall thickness of 0.5 mm and a surface area of 8, 000 mm². This packaging body 1 secures a good volatilization amount during a year, and the total volatilization amount in a year is in a range of about 2,600 to 3,000 mg with some errors taken into consideration.

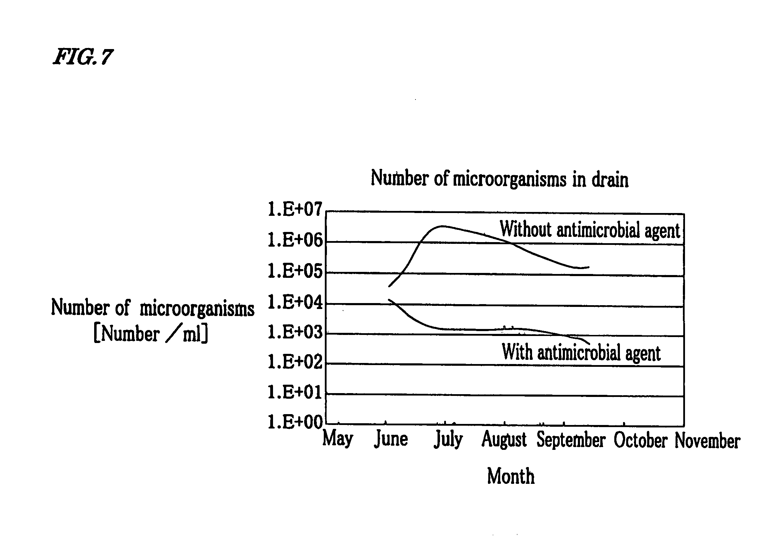

And, the number of microorganisms in drain of the air conditioner (with an antimicrobial agent) in which the packaging body 1 is disposed and that of microorganisms in drain of the air conditioner (without an antimicrobial agent) in which it is not disposed were compared. Fig. 7 shows the measured results. It is apparent from the drawing that, when the packaging body 1 of the embodiment was disposed, the number of microorganisms was considerably reduced. Thus, it was confirmed that the air conditioner was very effectively rendered antibacterial.

Then, a relationship among surface area X mm² of the casing 30, thickness Y mm of the wall section of the casing 30, and a volatilization amount of the volatile material will be described. Table 1 shows the measured values of volatilization amounts of the individual surface areas X when the wall section has thickness Y of 0.5 mm and 1.0 mm. And, Table 2 shows the measured values of Table 1 as the volatilization amounts to X/Y. Besides, Table 8 shows the measured values as a graph.

**[Table 1]**

| Surface area X | Volatilized amount per day with Y=0. 5 mm | Volatilized amount per day with Y=1.0 mm |
|---|---|---|
| 10000 [mm²] | 12.5 [mg/day] | 6.4 [mg/day] |
| 8000 | 10 | 5.1 |
| 6000 | 7.5 | 3.8 |
| 4000 | 4.9 | 2.5 |
| 2000 | 2.5 | 1.3 |

**[Table 2]**

| X/Y | Volatilized amount per day with Y=0. 5 mm | Volatilized amount per day with Y=1. 0 mm |
|---|---|---|
| 20000 | 12.5 [mg/day] | |
| 16000 | 10 | |
| 12000 | 7.5 | |
| 10000 | | 6.4 [mg/day] |
| 8000 | 4.9 | 5.1 |
| 6000 | | 3.8 |
| 4000 | 2.5 | 2.5 |
| 2000 | | 1.3 |

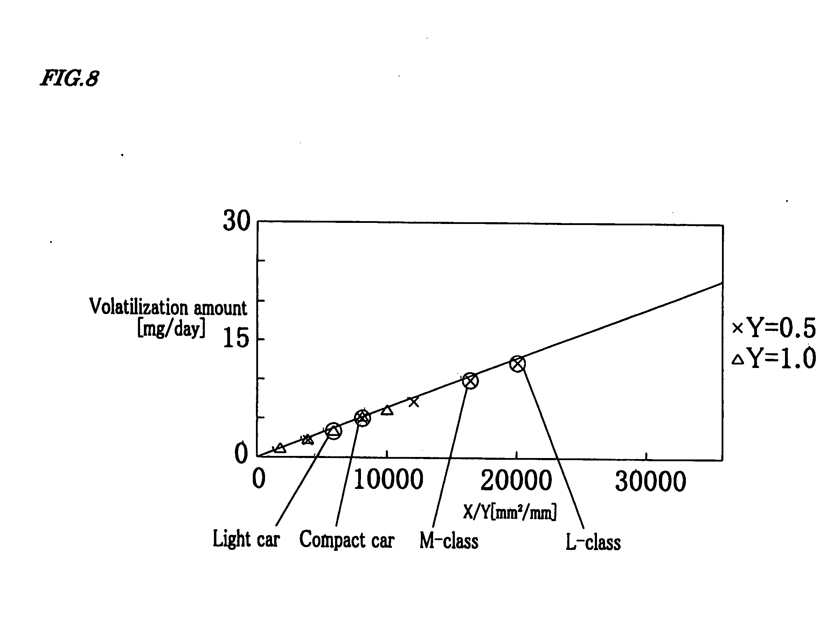

It is apparent from Fig. 8 that the volatilization amount of the volatile antimicrobial agent is substantially proportional to X/Y. Generally, an HVAC unit for a small car having a volume of about 100 L needs a volatilization amount of about 5.0 mg per day. And, it is considered in view of a small light-weight car that the lower limit of the range of the volatilization amount suitable for the car is about 3.8 mg per day. And, M-class and L-class cars require the volatilization amount of the volatile antimicrobial agent in a greater amount and the upper limit of the range of the volatilization amount suitable to such cars is considered to be about 17.0 mg per day. Specifically, a practical range of X/Y is 6,000 to 35,000, and a more desirable rang is 6,000 to 25,000 when the packaging body 1 is used to render car air conditioners antibacterial. When the wall thickness is not fixed, X/Y is calculated with an average of the entire thickness of the wall section determined as Y.

### [Effects of the Invention]

As described above, the present invention relates to the packaging body for the volatile material, comprising the bag having the volatile material sealed therein and having gas permeability and the casing which has the bag sealed therein, wherein the casing has its wall section at least partly made of a resin having gas permeability, and the volatile material permeates through the wall section to volatilize outside. By configuring as described above, the packaging body excelling in efficient volatilization of the volatile material can be obtained.

### [Description of the Reference Numerals]

- 1: Volatile material packaging body
- 20: Bag
- 30: Casing
- 31: Casing member
- 32: Lid member

## Claims

1. A packaging body for a volatile material, comprising a bag, which has, in use, the volatile material sealed therein and has gas permeability, and a casing which has the bag sealed therein, wherein the casing has its wall section at least partly made of a resin having gas permeability, and the volatile material permeates through the wall section to volatilize outside,
**characterized in that**
X/Y falls in a range of 6,000 to 35,000 when the gas-permeable wall section of the casing has a surface area of X mm² and a thickness of Y mm.

2. The packaging body for a volatile material according to claim 1, wherein a volatilization amount per day of the volatile material is 3.8 to 23.0 mg at 30°C.

3. The packaging body for a volatile material according to claim 1 or 2, wherein the wall section is made of polypropylene having a thickness of 0/3 to 2.2 mm.

4. The packaging body for a volatile material according to any one of claims 1 to 3, wherein the volatile material is allylisothiocyanate or a substance agent containing it.

## Patentansprüche

1. Packungskörper für ein flüchtiges Material, umfassend eine Tasche, die im Gebrauch das flüchtige Material darin versiegelt aufweist und eine Gasdurchlässigkeit hat, und ein Gehäuse, das die Tasche darin versiegelt aufweist, wobei der Wandbereich des Gehäuses zumindest teilweise aus einem eine Gasdurchlässigkeit aufweisenden Harz hergestellt ist, und das flüchtige Material durch den Wandbereich dringt, so dass es sich auf der Außenseite verflüchtigt,
**dadurch gekennzeichnet, dass**
X/Y in einen Bereich von 6000 bis 35000 fällt, wenn der gasdurchlässige Wandbereich des Gehäuses einen Oberflächenbereich von X mm² und eine Dicke von Y mm aufweist.

2. Packungskörper für ein flüchtiges Material nach Anspruch 1, bei dem die Verflüchtigungsmenge des flüchtigen Materials pro Tag 3,8 bis 23,0 mg bei 30°C ist.

3. Packungskörper für ein flüchtiges Material nach Anspruch 1 oder 2, bei dem der Wandbereich aus einem Polypropylen hergestellt ist und eine Dicke zwischen 0,3 und 2,2 mm aufweist.

4. Packungskörper für ein flüchtiges Material nach einem der Ansprüche 1 bis 3, bei dem das flüchtige Material Allylisothiocyanat oder ein Substanzstoff ist, der dieses enthält.

## Revendications

1. Corps d'emballage pour une matière volatile, comprenant un sac, dans lequel, en utilisation, la matière volatile est scellée et a une perméabilité au gaz, et un boîtier dans lequel le sac est scellé, où la section de paroi du boîtier est au moins partiellement faite en une résine disposant d'une perméabilité au gaz, et la matière volatile s'infiltre à travers la section de paroi pour se volatiliser à l'extérieur,
**caractérisé en ce que**
X/Y se trouve dans une gamme de 6000 à 35000 lorsque la section de paroi perméable au gaz du boîtier a une superficie de X mm² et une épaisseur de Y mm.

2. Corps d'emballage pour une matière volatile selon la revendication 1, dans lequel une proportion de volatilisation par jour de la matière volatile est de 3,8 à 23,0 mg à 30°C.

3. Corps d'emballage pour une matière volatile selon la revendication 1 ou 2, dans lequel la section de paroi est faite en polypropylène ayant une épaisseur de 0, 3 à 2, 2 mm.

4. Corps d'emballage pour une matière volatile selon l'une quelconque des revendications 1 à 3, dans lequel la matière volatile est un isothiocyanate d'allyle ou un agent de substance qui le contient.
